# EUROPEAN PATENT SPECIFICATION

(11) **EP 2 825 126 B1**
(45) Date of publication and mention of the grant of the patent: **02.08.2017**
(21) Application number: 13741831.5
(22) Date of filing: 18.03.2013
(51) Int. Cl.: A61F 2/00

(54) **A STRUCTURE OF ARTIFICIAL ENDO-URETHRAL SPHINCTER**
AUFBAU EINES KÜNSTLICHEN ENDOURETHRALSCHLIESSMUSKELS
STRUCTURE DE SPHINCTER ENDO-URÉTRAL ARTIFICIEL

(30) Priority: 17.03.2012 IT PI20120025
(43) Date of publication of application: 21.01.2015
(73) Proprietor: Pinzi, Novello, 55041 Lido di Camaiore LU (IT); Mazzocchi, Tommaso, 51017 Pescia (PT) (IT); Giuliani, Giuseppe, 51017 Pescia PT (IT)
(72) Inventor: Pinzi, Novello, 55041 Lido di Camaiore LU (IT); Mazzocchi, Tommaso, 51017 Pescia (PT) (IT); Giuliani, Giuseppe, 51017 Pescia PT (IT)
(74) Representative: Celestino, Marco
(86) International application number: PCT/IB2013/052156
(87) International publication number: WO 2013/144770

(56) References cited:
- WO-A1-00/30560
- WO-A1-2010/080021
- WO-A1-2011/073969
- DE-A1-102007 046 984
- US-B1- 6 464 999

## Description

### Field of the invention

The present invention relates to the medical field and, in particular, it relates to an artificial endo-urethral sphincter, for example to be implanted into patients who suffer from urinary incontinence.

### Background of the invention

Many types of artificial urethral sphincters are known for treating urinary incontinence. They are used when such remedies as the outpatient therapy, the pharmacological therapy and the pelvic re-education are not effective. The artificial urethral sphincters are used also as alternative to the traditional surgical techniques, which provide a reconstruction of the bladder support structures.

For the implant of urethral sphincters mini-invasive techniques exist, such as the TVT (Tension-free Vaginal Tape Procedure), or other techniques, described for instance in WO2005009293, or also in WO2011121591, which describes a device to be put into the vagina, having a distal portion to be positioned in a subvesical position and a proximal portion to be arranged in a suburethral position. The distal and proximal portions are configured for turning from a non-deformable condition to a deformable condition, and vice-versa. The device also comprises a mechanism for controlling the deformation, in order to apply a pressure to the urethra.

Other artificial endo-urethral sphincters are described in US2005187428, ES2343449, WO2006115225, US6638208, US2008015548, WO2005077301, WO2004037134, US6623421, WO0015140, WO0002499, US6193646, WO9901172, US6237623, EP0700668, DE102007046984. WO 2011/073969 describes a urological device comprising a urological valve and a plurality of leaflet valve members that have a region of mutual co-action. The valve has a configuration normally closed, in which the valve members are engaged at region of co-action, and an open configuration, in which the stopper members are separated in order to allow a flow of urine through the valve. The valve is configured for turning from the closed configuration to that open by applying a urological pressure.

US 6,464,999 B1 describes a urethral valve in implants or devices for controlling the urinary incontinence, which have a variable opening pressure responsive to changes of physiological parameters such as the increase of the abdominal pressure caused by coughs. DE 108007046984 describes an urethral valve with an electromagnetic control. Nevertheless, the above artificial sphincters have at least one of the following drawbacks:
- a sudden or impulsive action of the patient, such as a cough, an effort of any nature like when lifting weights, may increase the intravesical pressure up to opening the sphincter, which causes leakage of urine;
- a closure defect, or in any case a sealing defect, of the sphincter may cause a leakage of urine;
- they make it difficult or impossible the insertion of inspection and/or surgical medical equipment, or they damage the tightness after the insertion.

### Summary of the invention

Therefore, it is a feature of the invention to provide a structure of artificial endo-urethral sphincter that can prevent unwanted urine leakage.

It is a particular feature of the invention to provide such a structure that solves the tightness problems of the prior art, preventing the leakage of urine through the sphincter.

It is another particular feature of the invention to provide such a structure that prevents urine leakage in the case of an uncontrolled increase of intravesical pressure, due to coughs and efforts of other nature.

It is another feature of the invention to provide a structure of artificial endo-urethral sphincter that can be implanted in an outpatient procedure, without requiring any invasive surgical operation.

It is also a feature of the invention to provide a structure of artificial endo-urethral sphincter that allows, once implanted, the insertion of inspective or surgical medical equipment, in order not to preclude the patient from any medical examination or treatment.

It is also a feature of the invention to provide a structure of artificial endo-urethral sphincter that, in case of defect of a component, makes it possible to an easy change, for restoring the correct operation without surgery.

These and other objects are achieved by a structure of artificial endo-urethral sphincter comprising:
- at least one hollow rigid body, configured for being fixed within the walls of a urethral-bladder lumen of a patient, in particular for being fixed within the urethra walls, the hollow rigid body defining a longitudinal channel arranged to convey urine from an upstream section towards a downstream section;
- a valve element housed within the longitudinal channel;
- a closure means for causing the valve element to turn from a closed configuration, in which the urine cannot flow through the longitudinal channel, to an open configuration, in which the urine can flow through the longitudinal channel, and vice-versa.

According to the invention, the structure comprises a safety element arranged within the longitudinal channel of the hollow rigid body downstream of the valve element, wherein the safety element comprises a block means structured for turning from a block configuration to a release configuration, wherein the safety element is configured, in the block configuration, for preventing an unwanted flow of urine through the valve element and wherein, in the release configuration, the safety element is configured for enabling a free flow of urine that has flown through the valve element.

According to the invention, the closure means of the valve element comprises resilient walls that, in the closed configuration, are at a rest condition and, in the open configuration, are configured for being deformed due to an abdominal pressure increase caused by the patient, up to a predetermined opening pressure of the valve element.

In an exemplary embodiment, the safety element comprises a valve safety element serially arranged downstream of the valve element and, regardless of the upstream safety element, the block means of the valve safety element comprises resilient walls that, in the block configuration, are at a rest condition and, in the release configuration, are configured for being deformed due to an abdominal pressure increase caused by the patient, up to a predetermined release pressure of the valve safety element.

In a structure in which both the closure means and the safety means comprise resilient walls that can be deformed by increasing the abdominal pressure, the two valve elements cooperate to define the true opening pressure of the structure, which is normally set between the highest opening pressure between the two valve elements or the common opening pressure of the two valve elements, and the sum of these pressures.

Such a valve element allows the passage of an inspection or surgical medical equipment. In fact, it can be opened by pressing the lower face of the shell portion of the equipment, in such a way that the wings defined by the slits are spaced apart, and an opening is formed wide enough to allow such passage. Moreover, once the medical equipment has been applied through the valve, the original tightness is maintained, since the resilient walls of both valve elements are less likely to be damaged.

The release pressure of the valve safety element may be lower than the opening pressure of the valve element.

In an alternative embodiment, the release pressure of the valve safety element may be higher than the opening pressure of the valve element. This way, a safer seal can be obtained, due to the cooperation of the shell-shaped portions of both valve element to block the lumen.

In an exemplary embodiment, the valve element may have an opening pressure that is higher than the opening pressure of the valve safety element, in order to clear the longitudinal channel between both valve elements from the fluid, once the urine has been evacuated.

Preferably, the resilient walls comprise a shell portion equipped with at least one through slit, wherein:
- in the closed or block configuration, the shell portion has a convex shape on the bladder side, thus forming a diaphragm, such that a urine pressure acting upon the shell portion keeps the through slit closed;
- in the open or release configuration, the shell portion has a concave shape opposite to the convex shape, wherein the through slit is deformed and open, and is configured for enabling a flow of urine,

In the convex shape, the shell portion is configured for bearing the urine pressure up to an ultimate pressure selected between the release pressure and the opening pressure such that, upon exceeding the ultimate pressure, the shell portion collapses into the concave shape, and enables a passage of urine through the through slit. This way, the true opening pressure is higher than the nominal opening pressure of the valve element, which prevents urine leakage.

Advantageously, the shell portion has a cap shape that has a plurality of converging star-shaped slits, in particular said slits converging to a central point of the cap shape.

In other exemplary embodiments, the safety element comprises an abutment member that, in the block configuration of the safety element, is structured for preventing the valve element from turning from the closed configuration to the open configuration, and in the release configuration is structured for moving away from the valve element and enables the latter to open. This way, the true opening pressure of the valve element is substantially infinite as long as the safety element is in the block position, and returns to the value of the nominal opening pressure, which depends upon the material and upon the geometric features when the safety element is in the release configuration. This prevents the structure from being actuated by a sudden increase of the intravesical pressure, as in the case of coughs and of a patient's physical effort.

More in detail, in these exemplary embodiments, the safety element, which is movable within the hollow rigid body, is a safety slider configured for constraining the resilient deformation of the shell portion, so that the opening pressure becomes high enough to prevent the deformable portion, which may have a shell shape, from turning from the closed configuration to the open configuration if a sudden increase of the urine pressure occurs, as in the case of a cough or of any patient's physical effort.

Preferably, the safety element comprises a return elastic element, in particular a spring, which is configured for firmly keeping the safety element in the block configuration. More in detail, the return elastic element is arranged to move back to a position where it does not interfere with the deformation of the shell portion, once the opening pressure has been exceeded. In particular, the elastic constant of the spring can be selected to prevent the deformable valve element from turning from the closed configuration to the open configuration for any sudden urine pressure increase.

Advantageously, the safety element comprises a magnetically sensitive element at an own end opposite to the shell portion, said magnetically sensitive element configured such that, by arranging an external magnetic or electromagnetic element with a same polarity as the magnetically sensitive element, a magnetic coupling is formed between the external magnetic or electromagnetic element and the magnetically sensitive element, and the safety element turns to the release configuration.

In order to releasably fasten the structure to the urethral-bladder lumen, the hollow rigid body may be associated with a stent. In particular, the stent may comprise a plurality of resiliently radially compliant coils, or may be a tubular stent provided with shape memory flared end portions configured to fit the end portions of the urethral-bladder lumen. This way, the structure can be put into the urethral-bladder lumen by a tubular introducer body, within which the stent fits by radial compression. Once the introducer body has been extracted, the structure is released, and the coils or the flared end portions return to their own rest positions, thus fastening the valve body to the urethra and to the mouth of the bladder.

The apparatus comprising a fastening system, a hollow rigid body and a shell portion is a further preferred exemplary embodiment of the invention. The same type of magnetic coupling with a device operated from outside can be exploited for obtaining further preferred exemplary embodiments of the valve.

### Brief description of the drawings

The invention will be now shown with the following description of an exemplary embodiment thereof, exemplifying but not limitative, with reference to the attached drawings in which:
- Fig. 1 shows a structure, according to the invention, arranged in a urethral-bladder lumen;
- Figs. 2A-2C show sectional, partially sectioned, perspective partially sectioned views of a structure, according to the invention, comprising two shell valve elements;
- Figs. 2D-2F show the structure of Figs. 2A-2C in three consecutive opening steps of the valve apparatus;
- Fig. 3A-3C shows a diaphragm valve element as in the structure of Figs. 2A-2F, in an elevation view, in a plan view and in a perspective view;
- Figs. 4A to 8D show shell valve elements of another exemplary embodiments;
- Fig. 9 is a perspective view of a structure comprising a stent made as a same workpiece with the hollow rigid body;
- Fig. 10A is a perspective view of a structure that has a stent mounted to the hollow rigid body;
- Figs. 10B-10C are sectional views of the structure of Fig. 10A in a positioning configuration and in a use configuration;
- Figs. 10D-10E are a longitudinal sectional view and a partially sectioned perspective view of the valve apparatus of the structure of Fig. 10A;
- Figs. 11A-11B are sectional views of a structure equipped with a safety slider in the block configuration and in the release configuration;
- Figs. 11C-11E show the structure of Figs. 11A and 11B in a cross sectional view, in a partially sectioned view, and in a partially sectioned perspective view;
- Figs. 11F,14C,20E,21C,22D,23E show magnetic or electromagnetic elements outside of the body of the patient, associated respectively to the structures of Figs. 11B,14B,20D,21B,22B,23B;
- Figs. 12A-12B show the safety slider of the structure of Figs. 11A-11E in a perspective view and in an elevation side view;
- Fig. 13 is a partially sectioned perspective view of a structure, according to the invention, comprising fastening elements to be fastened to the inside walls of the urethral-bladder lumen;
- Figs. 14A-14B are longitudinal sectional views of the structure of Fig. 13, with the fastening elements in an introduction configuration and in a use configuration, respectively;
- Figs. 15A-15B are partial cross sectional views of a structure according to the invention, equipped with a stent comprising radially expansible coils, in an introduction configuration and in a use configuration;
- Figs. 16A-16B are partial cross sectional views of a structure according to the invention, with a different stent of a different type, in an introduction configuration and in a use configuration.

### Description of preferred exemplary embodiments

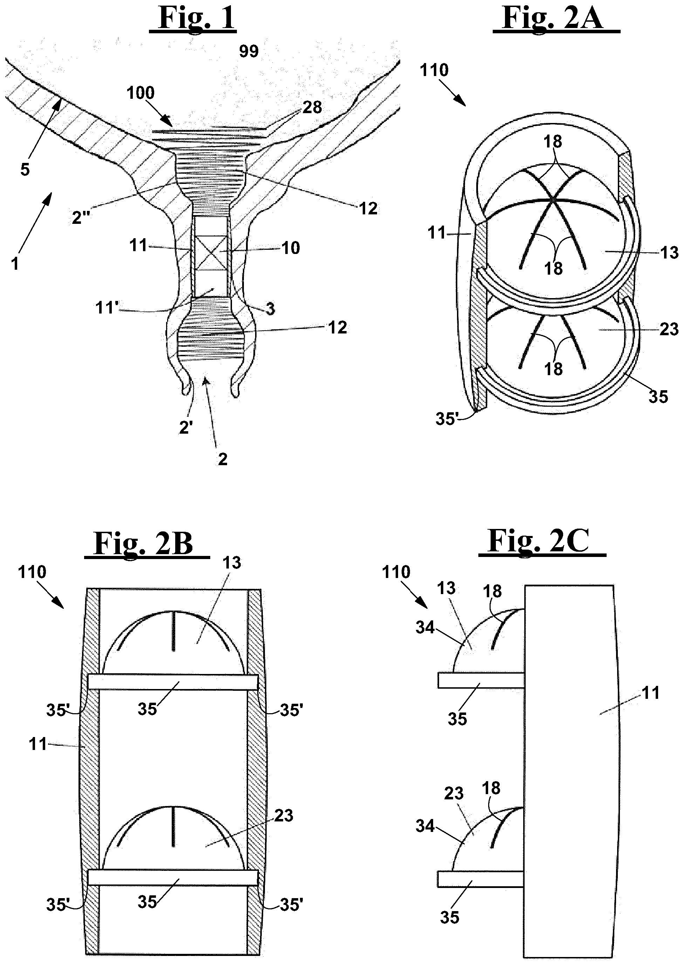

With reference to Fig. 1, a structure of artificial endo-urethral sphincter 100 is diagrammatically shown arranged within the urethral-bladder lumen 2 of a patient 1. Structure 100 comprises a hollow rigid body 11 that defines a longitudinal channel 11', and has an engagement means 12 to engage with inner walls 3 of urethral-bladder lumen 2 for fixing structure 100 within it.

In the case of Fig. 1, the engagement means may be provided by a stent 12. For instance, stent 12 may comprise coils 28 configured for radially expanding at end portions 2',2" of urethral-bladder lumen 2, as also shown in Figs. 17A-17B.

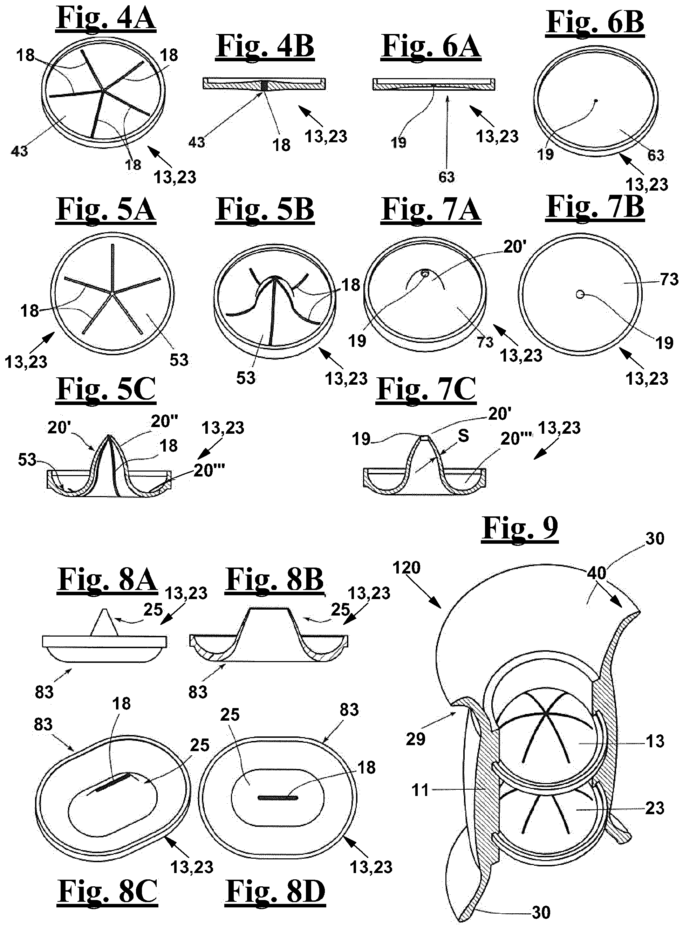

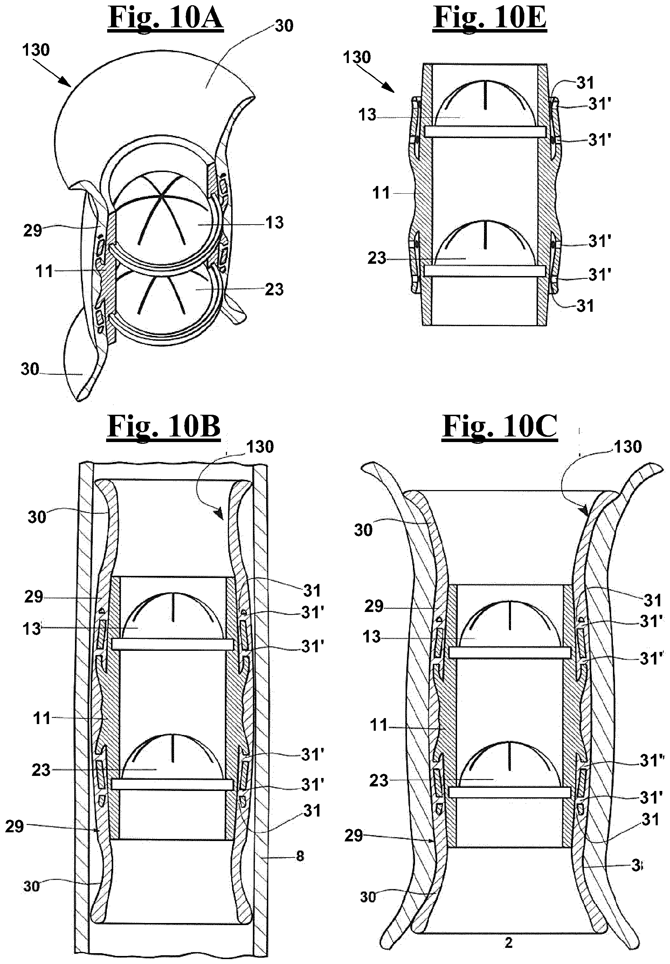

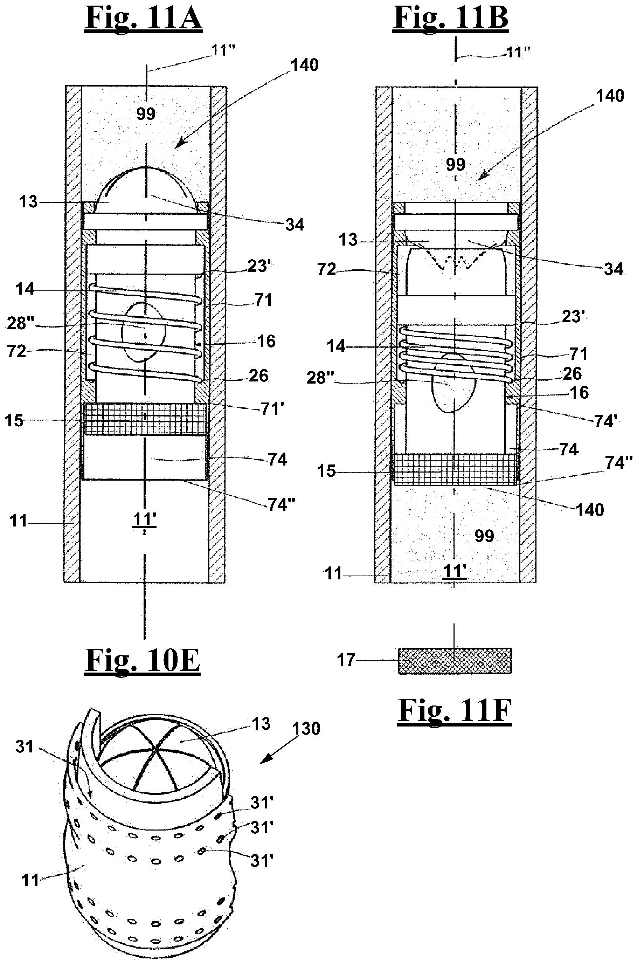

In alternative, in an exemplary embodiment, the structures 120,130 shown in Figs. 9 and 10A-C comprise a stent 29 that has flared shape memory end portions 30. In particular, in the structure 110 (Fig. 9), stent 29 is manufactured integral to hollow rigid body 11, whereas in the structure 110 (Figs. 10A-C) hollow rigid body 11 and stent 29 are two distinct bodies integrally connected to each other.

In Fig. 10B, structure 130 is shown in an introduction retracted configuration, where stent 29 fits within walls of a positioning device 8, such as a cannula, which can be put into urethral-bladder lumen 2 (Fig. 1). In Fig. 10C, structure 130 is shown in a use configuration, in which end portions 30 of stent 29 have recovered their own original shape, once it has been put into urethral-bladder lumen 2, ensuring a steady coupling with inner walls 3 thereof.

Stent 29 can be connected with respect to hollow rigid body 11 by a co-moulding technique. In order to assist the biocompatible material of stent 29, for example silicone rubber, to anchor on the structural material of hollow rigid body 11, the latter is provided with anchoring pockets 31 (Figs. 10A-10C), preferably also with holes 31' to cause the biocompatible material of stent 29 to flow into in anchoring pockets 31.

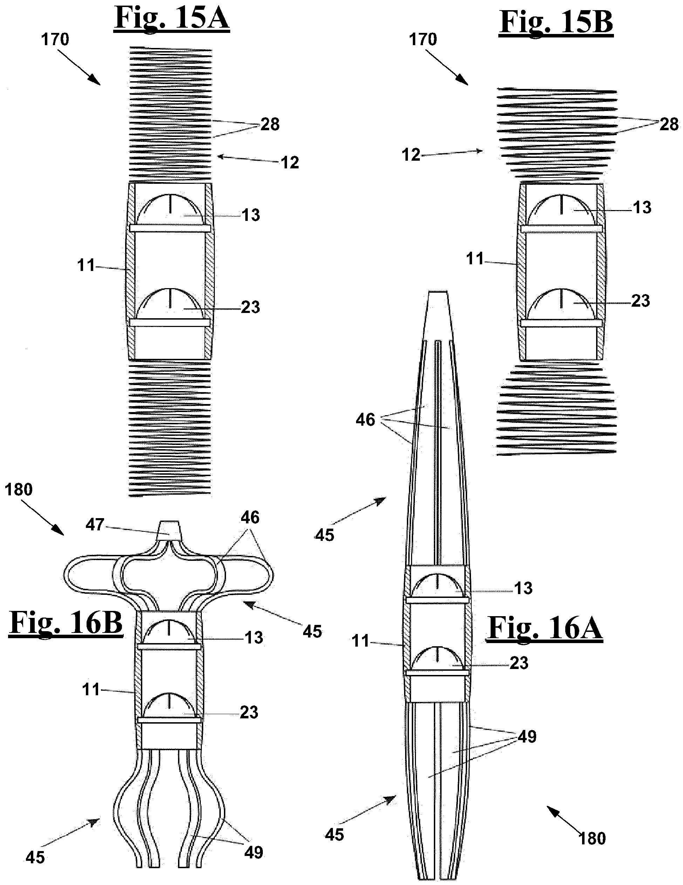

In alternative, in an exemplary embodiment, the structure 180 of Figs. 18A-18B comprises a stent 45 that has retractable shape memory longitudinal elements 46,49, which, in an extended configuration, allow the structure 180 to be introduced into urethral-bladder lumen 2 (Fig.16A), and after the introduction recover their own original retracted shape, thus a steady coupling with inner walls 3 of urethral-bladder lumen 2 (Fig.16B). Retractable elements 46, which are frontally arranged according to the insertion direction of structure 180 into the urethral-bladder lumen, preferably have their own end connected to a collection cuff 47.

As shown in Figs. 15 and 16A-16B, in an exemplary embodiment, the engagement means of a structure 160 may comprise fastening means 32 including fastening teeth 33 that are configured for engaging with inner walls 3 of urethral-bladder lumen 2 (Fig. 1). For instance, these fastening means may comprise a plurality of longitudinal elements 32 externally arranged along hollow rigid body 11, with fastening teeth 33 that protrude in a substantially radial direction with respect to hollow rigid body 11.

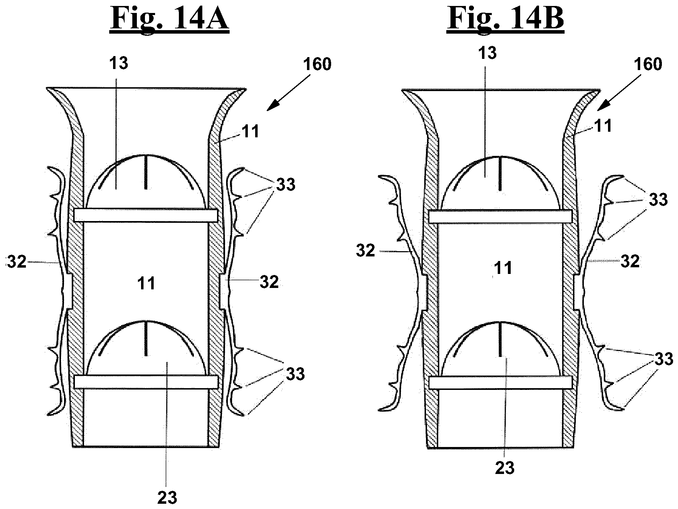

Fastening means 32 may be shape memory means, which turns from an introduction configuration (Fig. 14A) to an implant configuration (Fig.14B) during the implant.

Hollow rigid body 11, as well as engagement means 12,28,29,32,33,45, may be made of any biocompatible material, such as Teflon, titanium, PVC or polyurethane, since it comes directly into contact with the tissues of inner wall 3 of urethral-bladder lumen 2.

Still with reference to Fig. 1, the structure of artificial endo-urethral sphincter 100 has a valve apparatus 10 that is arranged within hollow rigid body 11 comprising a valve element 13,36 that is housed within longitudinal channel 11', shown more in detail in Figs. 2A-2F and 9-18. Valve apparatus 10 also comprises a closure means for causing valve element 13,36 to turn from a closed configuration, in which valve element 13,36 prevents urine 99, which is contained within bladder 5 of patient 1, from flowing through longitudinal channel 11', to an open configuration, in which urine 99 can flow through it, and vice-versa;

Figs. 2A-2F and 9-18 refer to exemplary embodiments according to the invention, in which valve apparatus 9 of the structure of endo-urethral sphincter 110,120,130,140,150,160,170,180 comprises a safety element 23,16 arranged within longitudinal channel 11' downstream of valve element 13,36. Safety element 23,16 comprises a block means that is structured to turn from a block configuration, in which it is configured for preventing an unwanted flow of urine 99 through valve element 13,36, to a release configuration, in which the safety element is configured for enabling a free flow of urine 99 that has flown through valve element 13,36.

A structure of artificial sphincter according to the invention may comprise two valve elements 13,23 or 16,23 serially arranged to each other in same hollow rigid body 11 or, according to an exemplary embodiment, not shown, in distinct rigid hollow bodies. Upstream valve element 13,36 is then a main valve element, or of control, whereas downstream valve element 23 is a valve safety element.

In particular, Figs. 2A-2F show a structure 110 in which the two valve elements are resilient deformable valve elements 13,23, each configured for turning from a closed configuration to an open configuration when the pressure of urine 99 exceeds a respective opening value, typically by an action of patient 1, such as an increase of the abdominal pressure. This opening value is indicated as the opening or release pressure of main valve element 13 and of valve safety element 23, respectively.

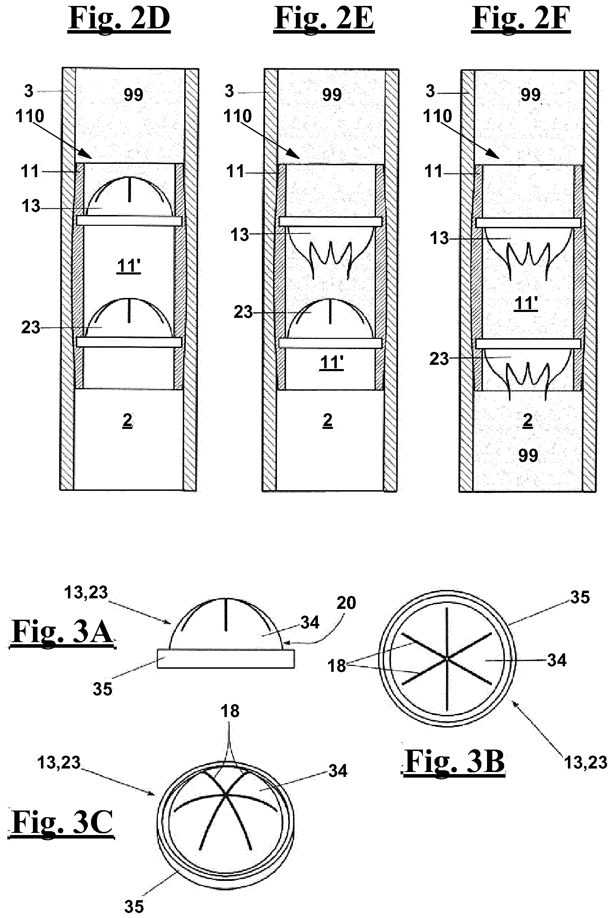

For example, valve elements 13,23 may be of the type shown in Figs. 3A-3C, in which resilient valve element 13,23 comprises a shell portion 34 and an engagement portion 35 for engaging with hollow rigid body 11. In particular, shell 34 has a cap, i.e. a convex portion 20 which may be substantially hemispherical or have the shape of a spherical sector. Shell 34 has at least one through slit 18, in particular a plurality of through slits 18 are made that converge towards a same portion of shell 34, preferably a top portion of cap 20.

In a version of structure 110, the release pressure of main valve element 23 is higher than the opening pressure of valve safety element 13.

In another version of structure 110, the release pressure of main valve element 23 is lower than the opening pressure of valve safety element 13.

Figs. 2D-2F show the operation of structure 110. Fig. 2D shows a rest condition of structure 110 in which the pressure of urine 99 is lower than the opening pressure of main valve element 13, and both valve elements 13,23 are in the closed configuration. Fig. 2E shows a condition in which valve elements 13 and 23 are in the open configuration and in the block configuration, respectively, the first under the action of the pressure of urine 99, which is higher than the opening pressure of main valve element 13 but is lower than the release pressure of valve safety element 23. Fig. 2F shows a condition in which both valve elements 13 and 23 are open, i.e. they are in the open configuration and in the release configuration, respectively, under the action of the pressure of urine 99, which is higher than both the opening pressure of valve element 13 and the release pressure of valve element 23.

The condition of Fig. 2E may take place in an initial step when normally using structure 110 to expel urine 99 from bladder 5, in which the pressure of urine 99 is progressively raised until it exceeds the opening pressure of the main valve element and then exceeds the release pressure of valve safety element 13, thus attaining the condition of Fig. 2F.

However, the condition of Fig. 2E make take place in the case of a sudden action of patient 1, such as a cough, or any efforts such as when lifting weights, which causes the urine pressure to exceed the opening pressure of main valve element 13 but not the exceeding the release pressure of safety element 23, so that valve element 23 remains in the block condition and prevents any unwanted outflow of urine through urethral-bladder lumen 2.

Still with reference to Fig. 2D and 2E, in the undeformed configuration shell portion 34 of valve element 13,23 has a convex portion in use oriented towards bladder 5. This way, urine 99 normally exerts a pressure on shell portion 34 that keeps slits 18 closed. By increasing the pressure of urine 18 the opening or release value, a critical stress condition of shell 34 is exceeded, beyond which the shell collapses and takes the deformed configuration of Fig. 2E, for upper valve element 13, and of Fig. 2F for both valve elements. In the deformed configuration, shell 34 has a convexity opposite with respect to the undeformed configuration, and slits 18 have their respective wings 18' spaced apart from one another, thus creating an opening in the shell 34. This opening allows urine 99 to massively flow through valve element 13,23.

Figs. 4A-8D show alternative embodiments of the resiliently deformable valve element 13,23. In particular, Figs. 4A,4B show a low curvature shell 43 that is provided with through slits 18 converging towards a central zone of the shell. Figs. 5A-5C show a shell 53 that has a convex cap portion 20', comprising opposite concavity portions 20",20''', as an alternative to spherical cap 20 of Figs. 3A-C, which also has through slits 18. As shown in Figs. 6A-6B and 7A-7C, in other exemplary embodiments, a shell 63,73 may have a suitably reamed hole 19, preferably made at the centre of the shell. Even in this case, the shell, in its undeformed configuration, may have a low curvature, like shell 63 of Figs. 6A-6B, or may have a cap portion 20', like shell 73 of Figs. 7A-7C. Figs. 8A-8D show a substantially ellipsoidal shell 83 that has a through slit 18 preferably at the peak of a cap portion 25 and/or oriented according to an axis of its elliptical cross section.

Shells 33,34,43,53,63,73 of Figs. 3A-8D are configured for resiliently collapse due to the absence of a stiff constraint member in their own central parts. This allows a quick deformation when the pressure of urine 99 on it passes the opening/releasing value, and allows, furthermore, a ready back stroke in the undeformed configuration when the pressure of urine 99 decreases below a corresponding closure/block value which, in particular, is lower than or the same as the opening/releasing value.

The ultimate opening pressure of resiliently deformable valve element 13,23 can be predetermined according to the shell stiffness and then flexural stiffness in the open configuration of shell 33,34,43,53,63,73,83. The stiffness, i.e. the resistance against the deformation, can be predetermined by choosing a predetermined thickness or thickness profile (for example, Fig. 7C), and/or by choosing a material that has predetermined elastic features, and/or by choosing other geometric features of the shell portion, for example the presence, the number, the size and the arrangement of slits 18, the presence and the shape of convex portion 20,20',25, of suitably reamed hole 19, in particular central of a central hole (Figs. 3A-8D).

The resiliently deformable valve element 13,23 comprises a stiff engagement portion 35 to engage with hollow rigid body 11, which may have form of a thick ring 35. In this case, hollow rigid body 11 has a circumferential inner groove 35' (Figs. 2A,2C) that serves as a housing for the engagement portion 35, in order to form a fluid-tight coupling. This allows a steady connection of valve element 13,23 with hollow rigid body 11, while allowing an opening/closing resilient deformation. Valve element 13,23, in particular its engagement portion 35, may be made of a resilient polymeric material such as polyurethane or silicone rubber, or may be made of a metal such as steel, Nitinol, titanium, or may be made of a different type of material, such as turbostratic carbon or graphite.

The shell portion, which is steadily mounted to the hollow rigid body by its own boundary portion, contributes specifically to the fluid-tightness of the sphincter. In a rest position, the shell portion is firmly closed, but it is opened once it has been deformed by increasing the urine pressure, under an arbitrary action of the patient. For instance, the shell portion can be opened by increasing the abdominal pressure.

The hollow rigid body is used to provide a dimensional stability to the structure as well as an internal fluid tightness. This requires a precise fluid-tight coupling between the hollow rigid body and the valve element or apparatus, along with a precise outer coupling between the hollow rigid body and the urethral-bladder lumen, in order to firmly engage with the latter.

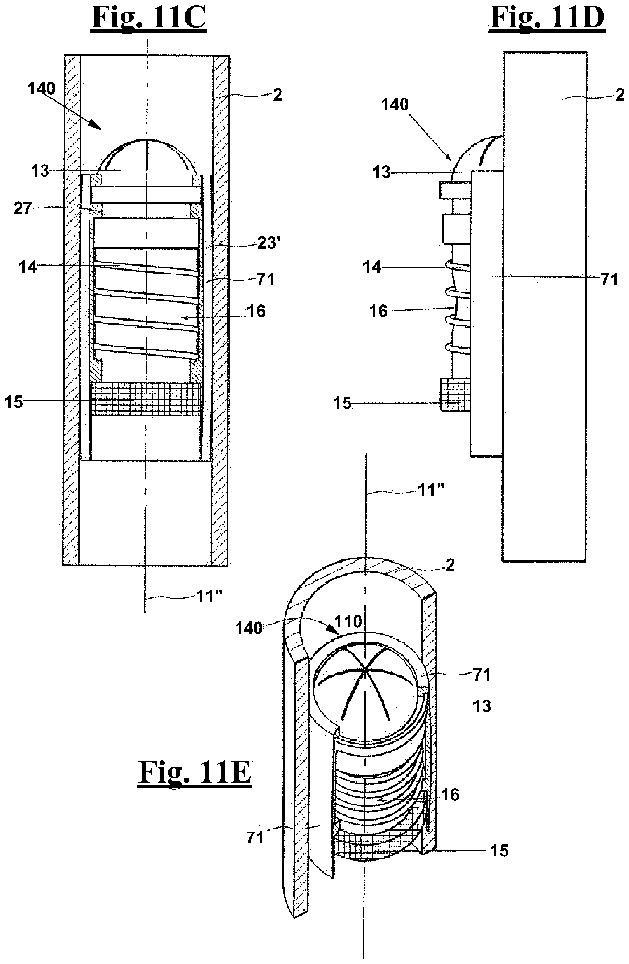

With reference to Figs. 11A-11E, a valve apparatus of a structure of artificial endo-urethral sphincter 140 comprises a resiliently deformable valve element 13, for example like in Figs. 3A-8D, and a safety element 16 that is movable within longitudinal channel 11'. Safety element 16 may have the shape of a safety slider 16 movably arranged between a block position (Fig. 11A) and a release position (Fig. 11 B). In the block position (Fig. 11A), safety slider 16 abuts against the face normally concave of shell 34 of valve element 13, i.e. against the face of shell 13 which, in use, is oriented opposite to bladder 5. In this position, slider 16 prevents valve element 13 from leaving the closed configuration. In the release position (Fig. 11 B) slider 16 is located at a prefixed distance from valve element 13, such that shell 34 is free to deform under the action of the pressure of urine 99, if this exceeds the opening pressure, then valve element 13 can leave the closed configuration and have the open configuration.

Valve apparatus 140 may also comprise a resilient return element 14 such as a return spring 14 that resists the movement of safety slider 16 from the block position (Fig. 11 A) to the release position (Fig. 11 B), so that safety slider 16, without any action from outside, is kept/brought in the block position of Fig. 11A. In the depicted shape, return spring 14 is wound about a preferably cylindrical portion of safety slider 16 and abuts, in the upper side, against a protruding portion 23' of safety slider 16 and, in the upper side, against an abutment portion 26 of hollow rigid body 71, that has an inner annular side 72 for housing spring 14 and slider 16.

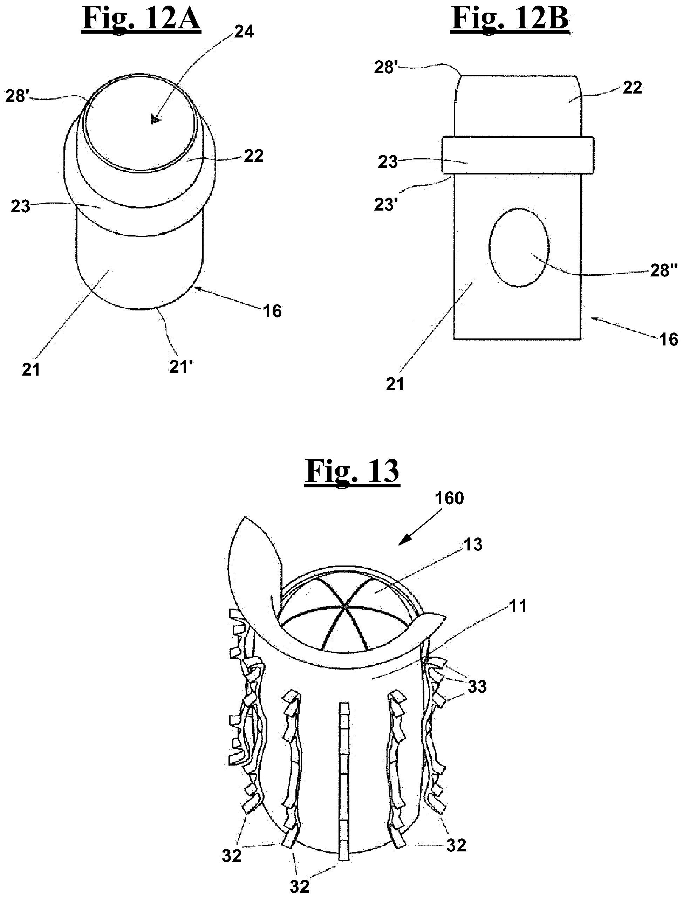

Safety slider 16 may have the shape shown in Figs. 12A-12B, which comprises a stem 21, a head 22 and an enlarged intermediate portion 23, and the three portions 21,22,23 have advantageously a substantially cylindrical shape. Head 22 is configured for engaging with shell portion 34 in the block position, provides a portion for winding spring 14 and the protruding face ring 23' of enlarged portion 23, which is oriented towards stem 21, provides the protruding portion against which abutment return spring 14 abuts. Advantageously, safety slider 16 comprises an inner passageway 24 between an upper opening 28', made through the portion facing valve element 13, and a lower opening 28", in the picture a side opening, made along stem 21, in order to let urine 99 when valve element 13 is in the open configuration under the pressure of urine 99 (Fig. 11 B).

Hollow rigid body 71 may also comprise an annular ring surface 27 (Fig. 11 C) of abutment for safety slider 16, with which enlarged portion 23 of slider 16 can abut when the safety slider is in the safety position.

Valve apparatus 140 may comprise a magnetically sensitive element 15 integral to safety slider 16 and arranged with a predetermined polarization direction, for example according to the direction of longitudinal channel 11', and an (electro)magnetic element 17 may also be provided outside of the body of the patient. Magnetically sensitive element 15 may be a permanent magnet or an element made of a ferromagnetic material. It may be arranged at the second free end 21' of stem 21, preferably on the opposite side with respect to valve element 13.

By arranging (electro)magnetic element 17 with a same polarity as magnet 15, safety slider 16 can be moved from block position (Fig. 11 A) back to the release position (Fig. 11 B), which allows the flow of urine 99 through main valve element 36.

By physiologically increasing the pressure of the urine, shell 34 can be collapsed and therefore valve element 13 can be opened, which enables urine 99 to flow out from bladder 5 (Fig. 1).

Magnetically sensitive ring 15 may be housed within a recess 74 of hollow rigid body 71 in which at least one upper end-stroke abutment surface 74',74" is preferably provided (Figs. 11A-11B).

In another exemplary embodiment, not shown, a valve apparatus comprises a deformable safety slider that is configured for radially collapsing under the action of a magnetic coupling transversal with respect to the axis of the valve apparatus, and also configured for resiliently recovering its own undeformed configuration, by removing the magnetic coupling, at which the safety slider keeps shell portion 34 in the undeformed configuration and, accordingly, valve element 13 remains closed.

Valve safety element 23 prevents urine leakage if valve element 13 accidentally opens, which may occur, for instance, in the case of a sudden action by patient 1, such as a cough, or any effort like when lifting weights, which may cause the pressure of urine 99 to exceed the opening pressure of valve element 13.

Obviously, even if engagement means 12,28,29,32,33,45 are always shown with reference to a structure of sphincter equipped with both resiliently deformable main and valve safety elements 13,23 (Fig. 2A), any type of engagement means can be used in other exemplary embodiments of the structure, such as the embodiment shown in Fig. 11A.

The foregoing description exemplary specific embodiments will so fully reveal the invention according to the conceptual point of view, so that others, by applying current knowledge, will be able to modify and/or adapt in various applications such exemplary embodiments without further research and without parting from the invention, and, accordingly, it is meant that such adaptations and modifications will have to be considered as equivalent to an exemplary embodiments exemplified. The means and the materials to realise the different functions described herein could have a different nature without, for this reason, departing from the field of the invention. It is to be understood that the phraseology or terminology that is employed herein is for the purpose of description and not of limitation.

## Claims

1. A structure (110,120,130,140,160,170,180) of artificial endo-urethral sphincter comprising:
- at least one hollow rigid body (11,71), configured for being fixed within the walls (3) of a urethral-bladder lumen (2) of a patient (1), in particular for being fixed within the urethral walls, said hollow rigid body (11,71) defining a longitudinal channel (11') arranged to convey urine from an upstream section towards a downstream section;
- a valve element (13) housed within said longitudinal channel (11');
- a closure means for causing said valve element (13) to turn from a closed configuration, in which said urine (99) cannot flow through said longitudinal channel (11'), to an open configuration, in which said urine (99) can flow through said longitudinal channel (11'), and vice-versa,
wherein said structure comprises a safety element (23,16) that is arranged within said longitudinal channel (11') of said hollow rigid body (11,71) downstream of said valve element (13),
wherein said safety element (23,16) comprises a block means structured for turning from a block configuration to a release configuration,
wherein said safety element (23,16) is configured, in said block configuration, for preventing an unwanted flow of urine (99) through said valve element (13),
and wherein said safety element is configured, in said release configuration, for enabling a free flow of urine (99) that has flown through said valve element (13),
**characterised in that** said closure means of said valve element (13) comprises resilient walls that, in said closed configuration, are at a rest condition and, in said open configuration, are configured for being deformed due to an abdominal pressure increase caused by said patient (1), up to a predetermined opening pressure of said valve element (13).

2. A structure (110,120,130, 160,170,180) according to claim 1, wherein said safety element comprises a valve safety element (23) serially arranged downstream of said valve element (13,36), and wherein said block means of said valve safety element (23) comprises resilient walls that, in said block configuration, are at a rest condition and, in said release configuration, are configured for being deformed due to an abdominal pressure increase caused by said patient (1), up to a predetermined release pressure of said valve safety element (23).

3. A structure according to claim 2, wherein said release pressure of said valve safety element (23) is lower than said opening pressure of said valve element (13).

4. A structure according to claim 2, wherein said release pressure of said valve safety element (23) is higher than said opening pressure of said valve element (13).

5. A structure according to claim 2, wherein said valve element (13) has an opening pressure that is higher than the opening pressure of the valve safety element (23), such that a portion of said longitudinal channel (11') defined between two valve elements (13,23) is cleared of said urine, once said urine (99) has been evacuated.

6. A structure (110,120,130,140,160,170,180) according to claim 1 or 2, wherein said resilient walls comprise a shell portion (34,43,53,63,73,83) that has at least one through slit (18,19), wherein:
- in said closed or block configuration, said shell portion (34,43,53,63,73,83) has a convex shape on the bladder side forming a diaphragm, such that a urine pressure acting upon said shell portion (34,43,53,63,73,83) keeps said through slit (18,19) closed;
- in said open or release configuration, said shell portion (34,43,53,63,73,83) has a concave shape opposite to said convex shape, wherein said through slit (18,19) is deformed and open, and is configured for enabling a flow of urine (99),
in said convex shape, said shell portion (34,43,53,63,73,83) configured for bearing said urine pressure (99) up to an ultimate pressure selected between said release pressure and said opening pressure such that, upon exceeding said ultimate pressure, said shell portion (34,43,53,63,73,83) collapses into said concave shape, and enables a passage of urine (99) through said through slit (18,19).

7. A structure (110,120,130,160,170,180) according to claim 6, wherein said shell portion (34,43,53,63,73,83) has a cap shape (20,20',25) that has a plurality of converging slits (18,19), in particular said slits converging to a central point of said cap shape.

8. A structure (140) according to claim 1, wherein said safety element comprises an abutment member (16) that, in said block configuration of said safety element (16), is structured for preventing said valve element (13) from turning from said closed configuration to said open configuration and, in said release configuration, is structured for moving away from said valve element (13) and enables the latter to open.

9. A structure (140) according to claim 8, wherein said safety element (16) comprises a return elastic element (14), in particular a spring (14), configured for firmly keeping said safety element (16) in said block configuration.

10. A structure (140) according to claim 8, wherein said safety element (16) comprises a magnetically sensitive element (15) at an own end (21,21') opposite to said shell portion (34), said magnetically sensitive element configured in such a way that, by arranging an external magnetic or electromagnetic element (17) with a same polarity as said magnetically sensitive element (15), a magnetic coupling is formed between said external magnetic or electromagnetic element (17) and said magnetically sensitive element (15), and said safety element (16) turns to said release configuration.

11. A structure (120,130,160,170,180) according to claim 1, wherein said hollow rigid body (11,71) is associated with a stent (12,29,45) configured to reversibly anchor said structure to said urethral-bladder lumen (2), said stent (12,29,45) selected from the group consisting of:
- a stent (12) comprising a plurality of resiliently radially compliant coils (28);
- a tubular stent (29,45) having shape memory flared (30,46,49) end portions.

## Patentansprüche

1. Struktur (110,120,130,140,160,170,180) eines künstlichen endourethralen Schließmuskels, umfassend:
- mindestens einen hohlen starren Körper (11,71), der konfiguriert, um innerhalb der Wände (3) eines urethralen Blasenlumens (2) eines Patienten (1) befestigt zu werden, besonders um innerhalb der urethralen Wände befestigt zu werden, wobei der hohle starre Körper (11,71) einen Längskanal (11') definiert, der angeordnet ist, um Harn aus einem vorherigen Abschnitt zu einem nachfolgenden Abschnitt zu leiten;
- ein Ventilelement (13), das in dem Längskanal (11') aufgenommen ist;
- ein Verschlussmittel, um zu veranlassen, dass das Ventilelement (13) von einer geschlossenen Konfiguration, in der der Harn (99) nicht durch den Längskanal (11') fließen kann, in eine offene Konfiguration zu drehen, in der der Harn (99) durch den Längskanal (11') fließen kann, und umgekehrt,
wobei die Struktur ein Sicherheitselement (23,16) umfasst, das in dem Längskanal (11') des hohlen starren Körpers (11,71) nach dem Ventilelement (13) angeordnet ist,
wobei das Sicherheitselement (23,16) ein Sperrmittel umfasst, das strukturiert ist, um von einer Sperrkonfiguration zu einer Freigabekonfiguration zu drehen, wobei das Sicherheitselement (23,16) in der Sperrkonfiguration konfiguriert ist, um einen unerwünschten Harnstrom (99) durch das Ventilelement (13) zu verhindern,
und wobei das Sicherheitselement in der Freigabekonfiguration konfiguriert ist, um einen freien Harnstrom (99) zu ermöglichen, der durch das Ventilelement (13) geflossen ist, **dadurch gekennzeichnet, dass** das Verschlussmittel des Ventilelements (13) elastische Wände umfasst, die in der geschlossenen Konfiguration in einem Ruhezustand sind, und in der offenen Konfiguration konfiguriert sind, um durch einen abdominalen Druckanstieg, verursacht durch den Patienten (1), bis zu einem vorbestimmten öffnungsdruck des Ventilelements (13) verformt zu werden.

2. Struktur (110,120,130,160,170,180) nach Anspruch 1, wobei das Sicherheitselement ein Ventilsicherheitselement (23) umfasst, das in Reihe nach dem Ventilelement (13,36) angeordnet ist, und wobei das Sperrmittel des Ventilsicherheitselements (23) elastische Wände umfasst, die in der Sperrkonfiguration in einem Ruhezustand sind und in der Freigabekonfiguration konfiguriert sind, um durch einen abdominalen Druckanstieg, verursacht durch den Patienten (1), bis zu einem vorbestimmten Freigabedruck des Ventilsicherheitselements (23) verformt zu werden.

3. Struktur nach Anspruch 2, wobei der Freigabedruck des Ventilsicherheitselements (23) niedriger als der Öffnungsdruck des Ventilelements (13) ist.

4. A Struktur nach Anspruch 2, wobei der Freigabedruck des Ventilsicherheitselements (23) höher als der Öffnungsdruck des Ventilelements (13) ist.

5. Struktur nach Anspruch 2, wobei das Ventilelement (13) einen Öffnungsdruck hat, der höher als der Öffnungsdruck des Ventilsicherheitselements (23) ist, sodass ein Abschnitt des Längskanals (11'), der zwischen zwei Ventilelementen (13,23) definiert ist, frei von dem Harn ist, nachdem der Harn (99) abgeleitet wurde.

6. Struktur (110,120,130,140,160,170,180) nach Anspruch 1 oder 2, wobei die elastischen Wände einen Mantelabschnitt (34,43,53,63,73,83) umfassen, der mindestens einen Durchgangsschlitz (18,19) hat, wobei:
- der Mantelabschnitt (34,43,53,63,73,83) in der geschlossenen oder Sperrkonfiguration eine konvexe Form auf der Blasenseite hat, die eine Membran bildet, sodass ein Harndruck, der auf den Mantelabschnitt (34,43,53,63,73,83) wirkt, den Durchgangsschlitz (18,19) geschlossen hält;
- der Mantelabschnitt (34,43,53,63,73,83) in der offenen oder Freigabekonfiguration eine konkave Form gegenüber der konvexen Form hat, wobei der Durchgangsschlitz (18,19) verformt und offen ist, und konfiguriert ist, um einen Harnstrom (99) zu ermöglichen,
der Mantelabschnitt (34,43,53,63,73,83) in der konvexen Form konfiguriert ist, um den Harndruck (99) auf einen ultimativen Druck zu bringen, der ausgewählt ist zwischen dem Freigabedruck und dem Öffnungsdruck, sodass bei Überschreiten des ultimativen Drucks der Mantelabschnitt (34,43,53,63,73,83) in die konkave Form kollabiert und einen Durchgang von Harn (99) durch den Durchgangsschlitz (18,19) ermöglicht.

7. Struktur (110,120,130,160,170,180) nach Anspruch 6, wobei der Mantelabschnitt (34,43,53,63,73,83) eine Haubenform (20,20',25) hat, die eine Vielzahl von konvergierenden Schlitzen (18,19) hat, wobei die Schlitze besonders zu einem Mittelpunkt der Haubenform konvergieren.

8. Struktur (140) nach Anspruch 1, wobei das Sicherheitselement ein Anschlagelement (16) umfasst, das in der Sperrkonfiguration des Sicherheitselements (16) strukturiert ist, um zu verhindern, dass das Ventilelement (13) von der geschlossenen Konfiguration zu der offenen Konfiguration dreht, und in der Freigabekonfiguration strukturiert ist, um sich vom Ventilelement (13) weg zu bewegen und ein Öffnen des Ventilelements zu ermöglichen.

9. Struktur (140) nach Anspruch 8, wobei das Sicherheitselement (16) ein elastisches Rückstellelement (14) umfasst, besonders eine Feder (14), die konfiguriert ist, um das Sicherheitselement (16) fest in der Sperrkonfiguration zu halten.

10. Struktur (140) nach Anspruch 8, wobei das Sicherheitselement (16) ein magnetisch empfindliches Element (15) an einem eigenen Ende (21,21') gegenüber dem Mantelabschnitt (34) umfasst, wobei das magnetisch empfindliche Element derart konfiguriert ist, dass durch Anordnen eines externen magnetischen oder elektromagnetischen Elements (17) mit einer gleichen Polarität wie das magnetisch empfindliche Element (15) eine magnetische Kopplung zwischen dem externen magnetischen oder elektromagnetischen Element (17) und dem magnetisch empfindlichen Element (15) gebildet wird, und das Sicherheitselement (16) in die Freigabekonfiguration dreht.

11. Struktur (120,130,160,170,180) nach Anspruch 1, wobei der hohle starre Körper (11,71) mit einem Stent (12,29,45) assoziiert ist, der konfiguriert ist, um die Struktur umkehrbar mit dem urethralen Blasenlumen (2) zu verankern, wobei der Stent (12,29,45) ausgewählt ist aus der Gruppe bestehend aus:
- einem Stent (12), der eine Vielzahl von elastisch radial nachgiebigen Spiralen (28) umfasst;
- einem rohrförmigen Stent (29,45) mit aufgeweiteten (30,46,49) Formgedächtnis-Endabschnitten.

## Revendications

1. Structure (110, 120, 130, 140, 160, 170, 180) de sphincter endo-urétral artificiel comprenant :
- au moins un corps rigide creux (11, 71), configuré pour être fixé dans les parois (3) d'une lumière urétro-vésicale (2) d'un patient (1), en particulier pour être fixé dans les parois urétrales, ledit corps rigide creux (11, 71) définissant un canal longitudinal (11') agencé pour transporter l'urine depuis une section en amont vers une section en aval ;
- un élément de valve (13) logé dans ledit canal longitudinal (11') ;
- un moyen de fermeture pour amener ledit élément de valve (13) à passer d'une configuration fermée, dans laquelle ladite urine (99) ne peut pas s'écouler à travers ledit canal longitudinal (11'), à une configuration ouverte, dans laquelle ladite urine (99) peut s'écouler à travers ledit canal longitudinal (11'), et vice-versa,
dans laquelle ladite structure comprend un élément de sécurité (23,16) qui est agencé dans ledit canal longitudinal (11') dudit corps rigide creux (11, 71) en aval dudit élément de valve (13),
dans laquelle ledit élément de sécurité (23, 16) comprend un moyen de blocage structuré pour passer d'une configuration de blocage à une configuration de libération, dans laquelle ledit élément de sécurité (23, 16) est configuré, dans ladite configuration de blocage, pour empêcher un écoulement indésirable d'urine (99) de traverser ledit élément de valve (13),
et dans laquelle ledit élément de sécurité est configuré, dans ladite configuration de libération, pour permettre un écoulement libre d'urine (99) qui s'est écoulée à travers ledit élément de valve (13),
**caractérisé en ce que** ledit moyen de fermeture dudit élément de valve (13) comprend des parois résilientes qui, dans ladite configuration fermée, sont dans une condition au repos et, dans ladite configuration ouverte, sont configurées pour être déformées du fait d'une augmentation de pression abdominale causée par ledit patient (1), jusqu'à une pression d'ouverture prédéterminée dudit élément de valve (13).

2. Structure (110, 120, 130, 160, 170, 180) selon la revendication 1, dans laquelle ledit élément de sécurité comprend un élément de sécurité de valve (23) agencé en série en aval dudit élément de valve (13, 36), et dans laquelle ledit moyen de blocage dudit élément de sécurité de valve (23) comprend des parois résilientes qui, dans ladite configuration de blocage, sont dans une condition au repos et, dans ladite configuration de libération, sont configurées pour être déformées du fait d'une augmentation de pression abdominale causée par ledit patient (1), jusqu'à une pression de libération prédéterminée dudit élément de sécurité de valve (23).

3. Structure selon la revendication 2, dans laquelle ladite pression de libération dudit élément de sécurité de valve (23) est inférieure à ladite pression d'ouverture dudit élément de valve (13).

4. Structure selon la revendication 2, dans laquelle ladite pression de libération dudit élément de sécurité de valve (23) est supérieure à ladite pression d'ouverture dudit élément de valve (13).

5. Structure selon la revendication 2, dans laquelle ledit élément de valve (13) présente une pression d'ouverture qui est supérieure à la pression d'ouverture de l'élément de sécurité de valve (23), de telle sorte qu'une partie dudit canal longitudinal (11') définie entre deux éléments de valve (13, 23) est vidée de ladite urine, une fois que ladite urine (99) a été évacuée.

6. Structure (110, 120, 130, 140, 160, 170, 180) selon la revendication 1 ou 2, dans laquelle lesdites parois résilientes comprennent une partie de coque (34, 43, 53, 63, 73, 83) qui présente au moins une fente traversante (18, 19), dans laquelle :
- dans ladite configuration fermée ou de blocage, ladite partie de coque (34, 43, 53, 63, 73, 83) présente une forme convexe du côté de la vessie formant un diaphragme, de telle sorte qu'une pression d'urine agissant sur ladite partie de coque (34, 43, 53, 63, 73, 83) maintient ladite fente traversante (18, 19) fermée ;
- dans ladite configuration ouverte ou de libération, ladite partie de coque (34, 43, 53, 63, 73, 83) présente une forme concave en regard de ladite forme convexe, dans laquelle ladite fente traversante (18, 19) est déformée et ouverte, et est configurée pour permettre un écoulement d'urine (99),
dans ladite forme convexe, ladite partie de coque (34, 43, 53, 63, 73, 83) configurée pour supporter ladite pression d'urine (99) jusqu'à une pression ultime sélectionnée entre ladite pression de libération et ladite pression d'ouverture de telle sorte que, au dépassement de ladite pression ultime, ladite partie de coque
(34, 43, 53, 63, 73, 83) s'affaisse dans ladite forme concave, et permet un passage d'urine (99) à travers ladite fente traversante (18, 19).

7. Structure (110, 120, 130, 160, 170, 180) selon la revendication 6, dans laquelle ladite partie de coque (34, 43, 53, 63, 73, 83) présente une forme de chapeau (20, 20', 25) qui présente une pluralité de fentes convergentes (18, 19), en particulier lesdites fentes convergeant vers un point central de ladite forme de chapeau.

8. Structure (140) selon la revendication 1, dans laquelle ledit élément de sécurité comprend un élément de butée (16) qui, dans ladite configuration de blocage dudit élément de sécurité (16), est structuré pour empêcher ledit élément de valve (13) de passer de ladite configuration fermée vers ladite configuration ouverte, et, dans ladite configuration de libération, est structuré pour s'éloigner dudit élément de valve (13) et permet à celui-ci de s'ouvrir.

9. Structure (140) selon la revendication 8, dans laquelle ledit élément de sécurité (16) comprend un élément élastique de retour (14), en particulier un ressort (14), configuré pour maintenir fermement ledit élément de sécurité (16) dans ladite configuration de blocage.

10. Structure (140) selon la revendication 8, dans laquelle ledit élément de sécurité (16) comprend un élément magnétiquement sensible (15) à une extrémité propre (21, 21') en regard de ladite partie de coque (34), ledit élément magnétiquement sensible configuré de telle sorte que, en agençant un élément magnétique ou électromagnétique externe (17) de même polarité que ledit élément magnétiquement sensible (15), un couplage magnétique soit formé entre ledit élément magnétique ou électromagnétique externe (17) et ledit élément magnétiquement sensible (15), et ledit élément de sécurité (16) passe dans ladite configuration de libération.

11. Structure (120, 130, 160, 170, 180) selon la revendication 1, dans laquelle ledit corps rigide creux (11, 71) est associé à un stent (12, 29, 45) configuré pour ancrer de manière réversible ladite structure à ladite lumière urétro-vésicale (2), ledit stent (12, 29, 45) sélectionné parmi le groupe constitué de :
- un stent (12) comprenant une pluralité de bobines compatibles radialement de manière résiliente (28) ;
- un stent tubulaire (29, 45) possédant des parties d'extrémité évasées à mémoire de forme (30, 46, 49).
